# EUROPEAN PATENT SPECIFICATION

(11) **EP 4 739 229 B1**
(45) Date of publication and mention of the grant of the patent: **11.11.2026**
(21) Application number: 24758345.3
(22) Date of filing: 05.07.2024
(51) Int. Cl.: A61B 17/22, A61B 17/3203, A61B 1/307, A61B 17/00, A61B 1/05

(54) **A CYSTOSCOPY DEVICE AND SYSTEM WITH A HYDRODYNAMIC CAVITATION PROBE**
ZYSTOSKOPIEVORRICHTUNG UND SYSTEM MIT EINER HYDRODYNAMISCHEN KAVITATIONSSONDE
DISPOSITIF ET SYSTÈME DE CYSTOSCOPIE AVEC SONDE DE CAVITATION HYDRODYNAMIQUE

(30) Priority: 07.07.2023 TR 202307953
(43) Date of publication of application: 13.05.2026
(73) Proprietor: Sabanci Üniversitesi, 34956 Tuzla/Istanbul (TR); Sabanci Üniversitesi Nanoteknoloji Arastirma ve Uygulama Merkezi Sunum, 34956 Tuzla/Istanbul (TR)
(72) Inventor: EKICI, Sinan, 34956 Istanbul (TR); GHORBANI, Morteza, 34956 Istanbul (TR); KUTLU, Ozlem, 34956 Istanbul (TR); KOSAR, Ali, 34956 Istanbul (TR); GOZUACIK, Devrim, 34956 Istanbul (TR)
(74) Representative: Genç Ilhan, Oznur
(86) International application number: PCT/TR2024/050770
(87) International publication number: WO 2025/014447

(56) References cited:
- EP-B1- 2 568 893
- WO-A1-2020/257397
- US-A1- 2020 337 548
- US-B2- 10 064 636

## Description

### Technical Field of the Invention

The invention relates to a cystoscopy device and system with a hydrodynamic cavitation probe.

### Background of the Invention

Cystoscopy is an endoscopic procedure that allows the examination of the inner surface of the bladder, the relationship between the prostate and the urinary tract, and the urethra (the tube that carries urine from the bladder out of the body). Cystoscopy is performed using a cystoscopy device through the urethra.

Cystoscopy devices can be used for diagnostic or therapeutic purposes. When a tissue sample is desired to be taken from the urinary system for these purposes, a surgical procedure is performed on the borders of the tissue sample to remove the tissue piece using cystoscopy devices. However, in cystoscopy devices prior to the art, surgical operations cannot be performed with the desired precision and sharp boundaries. This situation negatively affects the patient's treatment process.

New generation treatment methods comprise devices utilizing hydrodynamic cavitation. Surgical operations can be performed with the desired precision and sharp boundaries using hydrodynamic cavitation. The European patent publication number EP2568893B1 can be shown as an example of these devices.

A background art US 2020/337548 A1 discloses a leading-in device of a flexible ureteroscope and a using method thereof and a uretero-pyeloscope system. The leading-in device of the flexible ureteroscope includes a leading-in sheath component, the leading-in sheath component includes a leading-in sheath with elasticity, and the leading-in sheath is a strip-type tube body matched with the flexible ureteroscope. While the flexible ureteroscope is inserted and connected in the leading-in sheath, an outer end part of the flexible ureteroscope is aligned with an outer end part of the leading-in sheath or slightly protruded out of the outer end part of the leading-in sheath. The invention is capable of enabling the flexible ureteroscope to be accurately inserted into a renal pelvis, putting in place by one step, using conveniently, reducing injury of a patient, and reducing the cost of an operation.

The publication entitled "Design, Prototyping and Control of a Flexible Cystoscope for Biomedical Applications" from Canberk Sozer et al with reference "2017 IOP Conf. Ser.: Mater. Sci. Eng. 224 012050" discloses a cystoscope with a hydrodynamic cavitation probe and an articulated section having a plurality of joint units.

Due to the inadequacies of cystoscopy devices in the prior art, there is a need for a cystoscopy device and system with a hydrodynamic cavitation probe that allows surgical operations to create sharply defined tissue defects in the epithelial and subepithelial tissues after the surgical procedure.

### Objects of the Invention

The primary objective of the present invention is to develop a flexible cystoscopy device equipped with a hydrodynamic cavitation probe. As a result, a cystoscopy device based on hydrodynamic cavitation has been developed, allowing in-vivo surgical operations and ensuring that only sharply defined tissue defects occur in the epithelial and subepithelial tissues at the operation site after the operation.

Another objective of the invention is to develop a portable control unit for controlling and operating the mentioned cystoscopy device. A portable control unit, particularly manageable with one hand, contributes to the operator's efficient for use of the cystoscopy device.

Another objective of the invention is to develop a cystoscopy system comprising the mentioned cystoscopy device.

### Summary of the invention

The cystoscopy device according to the invention is defined in claim 1. Embodiments are defined in the dependent claims.

### Detailed Description of the Invention

A cavitation probe, being implemented to achieve the purpose of the invention, has been illustrated in the attached drawings wherein the details of the invention should be evaluated in view of the entire specification. These figures are:

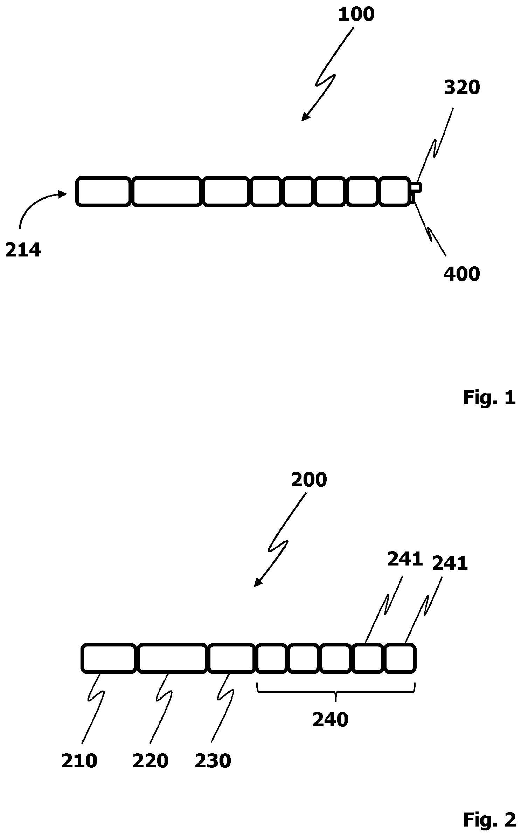

- **Fig 1.**: A schematic view of the cystoscopy device in one embodiment of the invention.
- **Fig 2.**: A schematic view of the main probe in one embodiment of the invention.

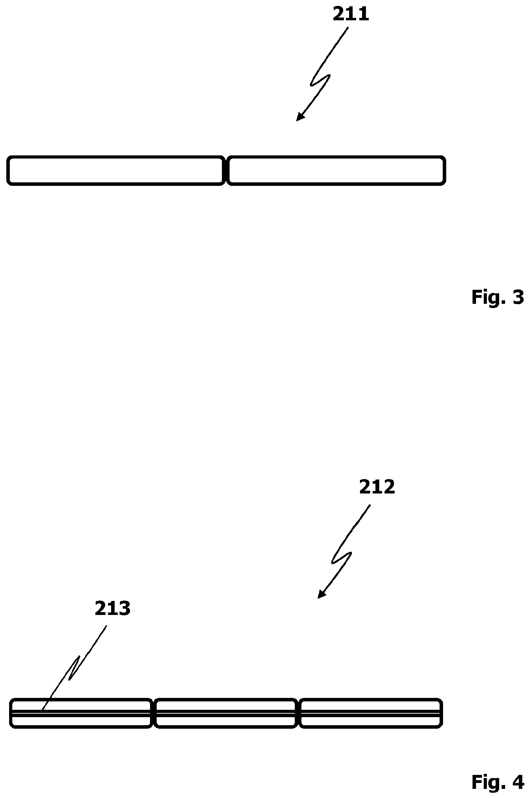

- **Fig 3.**: A schematic view of the two-part outer guide body in one embodiment of the invention.
- **Fig 4.**: A schematic view of the three-part inner guide body wherein the tendon channels are also shown in one embodiment of the invention.

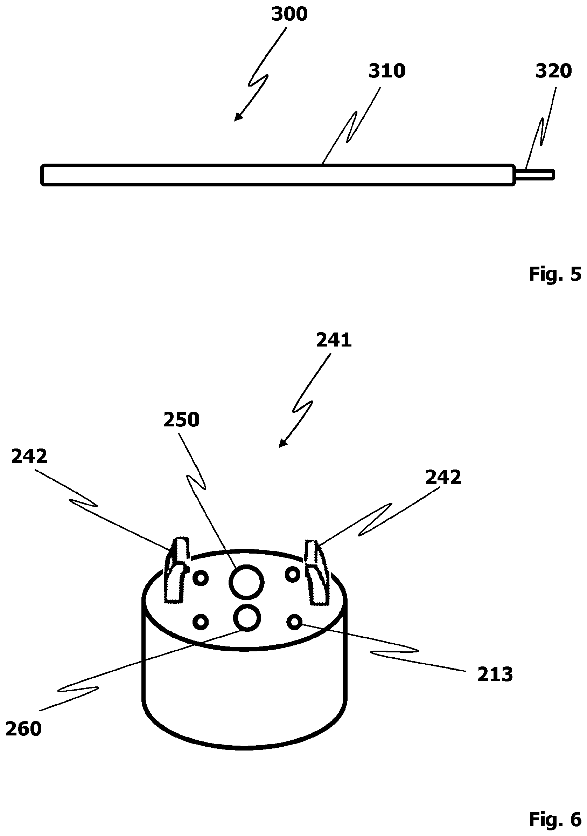

- **Fig 5.**: A schematic view of the cavitation probe in one embodiment of the invention.
- **Fig 6.**: A schematic view of a joint unit at the end surface of which connection threads are disposed in one embodiment of the invention.

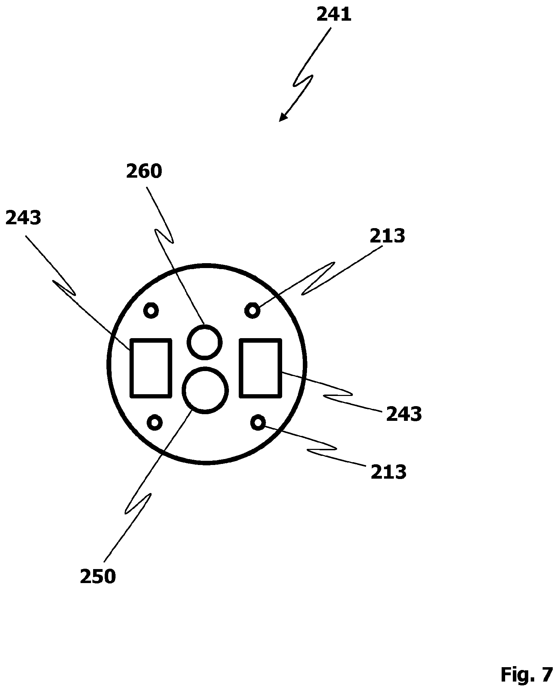

- **Fig 7.**: A schematic view of a joint unit at the end surface of which thread indentations are disposed in one embodiment of the invention.

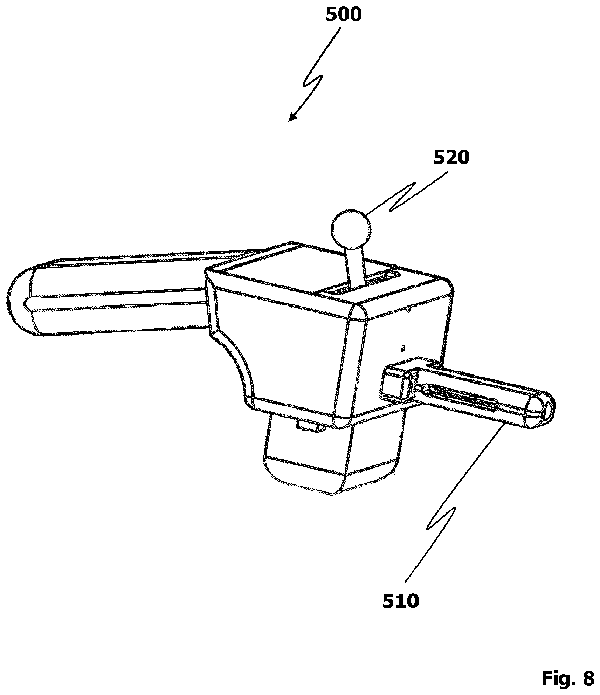

- **Fig 8.**: A schematic view of the portable control unit in one embodiment of the invention.

The reference numbers used in the drawings are given below.
- 100.: Cystoscopy device
- 200.: Main probe
210. Guide section
211. Outer guide body
212. Inner guide body
213. Tendon channel
214. Adapter inlet
220. Separation section
230. Abutment section
240. Articulated section
241. Joint unit
242. Connection tooth
243. Tooth recesses
250. Probe channel
260. Cable channel
- 300.: Cavitation probe
310. Transmission tube
320. Flow-restricting nozzle
- 400.: Image capturing optical unit
- 500.: Portable control unit
510. Probe adapter
520. Control lever

The invention relates to a cystoscopy device (100) comprising a tubular main probe (200), a cavitation probe (300), and an image capturing optical unit (400). The cavitation probe (300) is positioned within the main probe (200). The main probe (200) facilitates the navigation of the cystoscopy device (100) along the application path during in-vivo procedures. The image capturing optical unit (400) is used to visualize the application path during the procedure. Thus, the operator directs the cystoscopy device (100) using the image taken from the application path via the image capturing optical unit (400) and positions the cystoscopy device (100) in the desired position in the application path. The cystoscopy device (100) comprises biocompatible materials due to its nature. In the embodiments of invention, the caliber size of the cystoscopy device (100) is preferably about 7 mm or less.

The main probe (200) sequentially comprises a guide section (210); a separation section (220); an abutment section (230); and an articulated section (240) having a plurality of joint units (241) aligned end to end.

The guide section (210) comprises at least one tubular outer guide body (211); at least one tubular inner guide body (212) positioned inside the outer guide body (211). A transmission tube (310) of the cavitation probe (300) and at least one connection cable electrically connected to the image capturing optical unit (400) pass through inside the inner guide body (212). Multiple tendon channels (213) extend along the inner guide body (212). Tendon channels (213) run between the inner guide body (212) and the outer guide body (211). In one embodiment, the tendon channel (213) is a groove formed on the outer surface of the inner guide body (212). At the open end of the guiding section (210) (the end not connected to the separation section (220)), there is an adapter inlet (214) for accommodating a portable control unit (500) to the cystoscopy device (100). Through the adapter inlet (214), the portable control unit (500) is connected to the cystoscopy device (100). This enables the operator to control the main probe (200) and especially the bending movement of the articulated section (240).

The separation section (220) of the main probe (200) comprises at least one probe channel (250) for the transmission tube (310) extending from the inner guide body (212), at least one cable channel (260) for the connection cable and multiple tendon channels (213). The separation section (220) provides separate paths (channels: probe channel (250) and cable channel (260)) for the transmission tube (310) and the connection cable. Thereby, the transmission tube (310) and the connection cable are prevented from tangling or getting damaged during the movement of the main probe (200) and especially of the articulated section (240).

The abutment section (230) of the main probe (200) provides an abutting surface for the nearest joint unit (241) during the bending movement of the articulated section (240). The abutment section (230) comprises at least one probe channel (250) and at least one cable channel (260).

Each joint unit (241) of the articulated section (240) in the main probe (200) comprises at least one probe channel (250) and at least one cable channel (260). Multiple tendon cables extend along the tendon channels (213) in the guiding section (210), separation section (220), abutment section (230), and articulated section (240). One of the end surfaces of the joint units (241) aligned end to end has an inner concave form allowing movement in only one degree of freedom. The other end surface has an outer convex form to match the inner concave surface, allowing movement again in the only one degree of freedom. Each joint unit (241) positioned between two joint units (241) comprises at least two connection teeth (242) extending by facing one another on one end surface of each joint unit (241). The connection teeth (242) are in a concave form center of which faces the center of the central joint unit (241). At least two tooth recesses (243) are located on another end surface of the joint unit (241) for accommodating and allowing the movement of the connection teeth (242) inside the tooth recesses (243). The connection teeth (242) (together with the tooth recesses (243)) connect the joint units (241) to each other and ensure that the joint units (241) do not separate from each other during their movements relative to one another. The connection teeth (242) limit the movement of adjacent joint units (241) relative to each other. The freedom of movement of adjacent joint units (241) relative to each other (the degree of angular bending) varies depending on the concave and convex angles of the end surfaces of the connected joint units (241) as well as the concave angles and sizes of the connection teeth (242). In an exemplary embodiment of the invention, the articulated section (240) comprises five joint units (241) positioned end-to-end. In the exemplary embodiment, each joint unit (241) has a movement freedom of approximately 8° relative to each other. Thus, the articulated section (240) can perform a bending movement with a total freedom degree of 80°, 40° in one direction and 40° in the opposite direction.

In one embodiment, the connection teeth (242) are located on the convex surface, and the tooth recesses (243) are on the concave surface of the joint units (241).

The cavitation probe (300) comprises a flexible transmission tube (310) extending along the main probe (200) for transmitting a cavitation fluid to a flow-restricting nozzle (320). The flow-restricting nozzle (320) is located at one end of the transmission tube (310). In an exemplary application, the flow-restricting nozzle (320) has a tubular structure. The flow-restricting nozzle (320) has an internal radius smaller than the internal radius of the transmission tube (310) for creating, in order to create a cavitation formation. The flow-restricting nozzle (320) is located at an open end of the articulated section (240) (at the last joint unit (241), which is one end of the main probe (200) where the adapter inlet (214) is not disposed).

The image capturing optical unit (400) is located at the open end of the articulated section (240) (at the last joint unit (241), which is one end of the main probe (200) where the adapter inlet (214) is not disposed).

In an embodiment of the cystoscopy device (100) comprises a portable control unit (500) with a probe adapter (510) for being accommodated into the adapter inlet (214). In an embodiment of the invention, the probe adapter (510) and the adapter inlet (214) are coupled/interfaced together. Thus, the tendon cables of the cystoscopy device (100) are mechanically connected to the portable control unit (500). [Multiple tendon cables run along the tendon channels (213) of the guide section (210), the separating section (220), the abutment section (230), and the articulated section (240).] The portable control unit (500) comprises at least one drive unit for changing the tension in the tendon cables (to apply a pulling force to the tendon cables). By changing the tension in the tendon cables, the articulated section (240) can perform a bending movement with only one degree of freedom. The portable control unit (500) comprises a control lever (520) to control the drive unit (to determine how much the drive unit will change the tension in the tendon cables and how much pulling force will be applied to the tendon cables) and thus to control the bending movement of the articulated section (240). This control lever (520) is preferably designed to be controlled with a single finger (for example, a thumb control lever (520) (a control lever (520) in the form of a ring through which the thumb can pass)). With the control lever (520), the operator can gradually adjust how much the drive unit will change the tension in the tendon cables and how much pulling force will be applied to the tendon cables. In a variation of this embodiment, the drive unit comprises a servo motor and a servo arm attached to the servo motor at one part and connected to the tendon cables at its ends. The rotational movement of the servo motor also causes the servo arm to rotate. The tendon cables connected to the ends of the servo arm are tensioned in the direction of rotation. Thus, the bending movement of the articulated section (240) in only one direction (for example, to the right or left) with one degree of freedom occurs in the direction of tension/pulling of the tendon cables. In another variation of this embodiment, the portable control unit (500) comprises a processing unit. The processing unit is adapted to create a selected (continuous) tension in the tendon cables via the servo motor to maintain the position of the articulated section (240) in a position adjusted by the control lever (520). The operator positions the articulated section (240), especially the open end of the articulated section (240) (where the flow-restricting nozzle (320) and the image capturing optical unit (400) are located) via the control lever (520). To keep the open end of the articulated section (240) stable in one position (without shaking or position change), the tendon cables need to remain at a selected tension. The processing unit provides a selected tension to the tendon cables via the servo motor to ensure stability (being stable in one position) according to the position.

In one embodiment of the invention, the joint unit (241) located at the open end of the articulated section (240) (the outermost joint unit (241)) has an inverted U-shaped surface structure on its outermost surface. The surface structure forms a bearing for a tendon cable. The surface structure is located between two tendon channels (213). The tendon cable emerging/exiting from one channel of the joint unit (241) is guided (passes over) the inverted U-shaped surface structure (from the bottom of the U) and transitions to another tendon channel (213). From here, the tendon cable extends through the tendon channels (213) of the articulated section (240), the abutment section (230), and the separating section (220), and then through the inner guide body (212) of the guide section (210) to the adapter inlet (214). Thus, it is sufficient to use two tendon cables instead of four to achieve the bending movement of the articulated section (240). Moreover, the need for fixing units necessary for securing the tendon cables to the open end of the articulated section (240) is eliminated. This helps to ensure the position stability of the articulated section (240), reduce the size of the cystoscopy device (100), make it lighter, and extend its service life.

In one embodiment of the invention, the cavitation probe (300) is made of a material comprising polyether ether ketone (PEEK) polymer. Preferably, PEEK polymer material is used for both the transmission tube (310) and the flow-restricting nozzle (320). The transmission tube (310) must be flexible to be in conformity with the bending movements of the articulated section (240). The transmission tube (310) must also be highly resistant to pressure to transmit the cavitation fluid to the flow-restricting nozzle (320) at high pressures. Studies have shown that the PEEK polymer transmission tube (310) can continuously operate for at least 3 hours at a pressure of 450 psi (3102 kPa).

In one embodiment of the invention, the main probe (200) and the cavitation probe (300) are modular. To enable the cystoscopy device (100) to perform operations, for example, to reach organs in the urinary system, it must be longer than a certain length. This length exceeds the dimensions that a traditional 3D printer (such as DMLS) can create. With this application, the production of the cystoscopy device (100) with a 3D printer is made possible. In this embodiment, the outer guide body (211) and the inner guide body (212) have multiple tubular bodies joined end to end.

The application comprises a cystoscopy system containing any embodiment of the cystoscopy device (100). The cystoscopy system comprises a fluid system to transmit a cavitation fluid to the cavitation probe (300) at a selected pressure and/or at selected time intervals, and an imaging system electrically connected to the image capturing optical unit (400) via a connection cable to display the image captured in the image capturing optical unit (400). In one embodiment of the invention, the fluid system comprises a high-pressure nitrogen tank, a cavitation fluid reservoir, a fluid control valve, a one-way valve, a filter, and a pressure gauge.

One of the biggest problems with cystoscopy devices in prior art is their caliber size. High caliber size prevents the use of cystoscopy devices (100) in many patients (for example, pediatric patients). The cystoscopy device (100) of the present application comprises many technical features to reduce caliber size. These features are particularly found in the construction of the main probe (200), especially the articulated section (240) and the portable control unit (500). To reduce caliber size, the articulated section (240) is limited to perform a bending movement with only one degree of freedom. The movement disadvantage brought by this limitation is compensated by the implementation of a portable control unit (500) instead of a conventional desktop control unit. With the help of the portable control unit (500), the operator can provide the articulated section (240) with a second degree of freedom by a yaw (rotation around its axis) movement. Another feature to reduce caliber size is the inverted U-shaped surface structure. By using the inverted U-shaped surface structure, the movement of the articulated section (240) is achieved using 2 tendon cables instead of four. The surface structure allows for the creation of a smaller caliber cystoscopy device (100) by reducing the number of tendon cables used. Moreover, the need for fixing units necessary for securing the tendon cables to the open end of the articulated section (240) is eliminated, helping to reduce the caliber size. The reduction in caliber size of the articulated section (240) also brings the necessity to reduce the caliber size of the cavitation probe (300) (since it passes through the articulated section (240)). The material to be used for the cavitation probe (300) must also be highly resistant to pressure, flexible, and durable. Therefore, in one embodiment of the invention, a cavitation probe (300) made of PEEK polymer material is used. Thus, a cystoscopy device (100) capable of performing operations at high pressures for a long time with a small caliber has been realized.

An exemplary embodiment of the invention is as follows:
The operator positions the cystoscopy device (100), especially the articulated section (240), in the region where the operation will be performed. The surgical operation is performed with hydrodynamic cavitation created with the help of the flow-restricting nozzle (320) located at the open end of the articulated section (240). During the operation, the operator changes the position of the flow-restricting nozzle (320) with the help of the portable control unit (500) to complete the local invasive operation. Here, the hydrodynamic cavitation occurs as follows. The cavitation fluid advancing through the transmission tube (310) reaches the flow-restricting nozzle (320). The inner radius of the flow-restricting nozzle (320) is smaller than the inner radius of the transmission tube (310). Due to the sudden decrease in radius in the cross-sectional area where the cavitation fluid progresses, the fluid velocity increases according to Bernoulli's principle. As the fluid velocity increases, the static pressure of the fluid falls below the vapor pressure, and cavitation bubbles begin to form due to vaporization. When a cavitation fluid jet, emerging from the end of the flow-restricting nozzle (320), strikes the tissue samples, the cavitation bubbles within the jet burst near the tissue. The stored energy in the bubbles affects the tissue along with the re-entrant jets and shock waves. The formation of re-entrant jets occurs when the cavitation bubble reaches its maximum volume and strikes the tissue surface at speeds of up to several hundred meters per second. Subsequently, a water hummer shock occurs as a result of the effect of the re-entrant jet on the distal part of the bubble, leading to the formation of primary shock waves. Then, the cavitation bubble suddenly collapses, producing a secondary shock wave. Due to the interaction of primary and secondary shock waves, high-pressure areas between the tissue and bubbles cause the cavitation bubbles in the fluid jet near the tissue to collapse forcefully, resulting in physical damage to the surface of the target tissue.

## Claims

1. A cystoscopy device (100) comprising a tubular main probe (200); a cavitation probe (300); an image capturing optical unit (400) wherein the main probe (200) sequentially comprises a guide section (210); a separation section (220); an abutment section (230); and an articulated section (240) having a plurality of joint units (241) aligned end to end;
wherein the guide section (210) comprises at least one tubular outer guide body (211); at least one tubular inner guide body (212) positioned inside the outer guide body (211) through which a transmission tube (310) of the cavitation probe (300) and at least one connection cable electrically connected to the image capturing optical unit (400) pass; a plurality of tendon channels (213) extending between the inner guide body (212) and the outer guide body (211) along the inner guide body (212); and an adapter inlet (214) at its open end for accommodating a portable control unit (500);
wherein the separation section (220) comprises at least one probe channel (250) for accommodating the transmission tube (310) extending from the inner guide body (212); at least one cable channel (260) for accommodating the connection cable; and a plurality of tendon channels (213);
wherein the abutment section (230) is for providing an abutting surface for the nearest joint unit (241) of the articulated section (240) during a bending movement; wherein each joint unit (241) comprises at least one probe channel (250) and at least one cable channel (260); wherein one of the end surfaces of the joint units (241) aligned end to end has an inner concave form allowing movement in only one degree of freedom and another end surface has an outer convex form to match the inner concave surface; wherein each joint unit (241) positioned between two joint units (241) comprises at least two connection teeth (242) extending by facing one another on one end surface, limiting the relative movement of the neighboring joint units (241) and center of which face the center of the central joint unit (241); at least two tooth recesses (243) on the other end surface for accommodating and allowing the movement of the connection teeth (242) inside; wherein a plurality of tendon cables extend along the tendon channels (213) of the guide section (210), separation section (220), abutment section (230), and articulated section (240);
wherein the cavitation probe (300) comprises a flexible transmission tube (310) extending along the main probe (200) for transmitting a cavitation fluid to a flow-restricting nozzle (320); the flow-restricting nozzle (320) positioned at an open end of the articulated section (240) having an inner radius smaller than the inner radius of the transmission tube (310) to create cavitation;
wherein the image capturing optical unit (400) is positioned at the open end of the articulated section (240).

2. A cystoscopy device (100) according to claim 1, comprising the portable control unit (500) having a probe adapter (510) for being accommodated into the adapter inlet (214); wherein the portable control unit (500) comprises at least one drive unit for changing the tension in a plurality of tendon cables extending along the tendon channels (213) and thereby allowing the articulated section (240) to bend and move in only one degree of freedom; and a control lever (520) for controlling the drive unit and thereby controlling the bending movement of the articulated section (240).

3. A cystoscopy device (100) according to claim 2, wherein the drive unit is a servo motor; and comprising a servo arm connected to the servo motor on one end and to the tendon cables on the other end.

4. A cystoscopy device (100) according to any of claims 2 or 3, comprising a processing unit adapted to create a selected tension in the tendon cables via the servo motor to maintain the position of the articulated section (240) in a position adjusted by the control lever (520).

5. A cystoscopy device (100) according to any of the preceding claims, comprising an inverted U-shaped surface structure on the surface of the joint unit (241) positioned at the open end of the articulated section (240), between two tendon channels (213), through which a tendon cable exiting one tendon channel (312) is guided to the other tendon channel (213).

6. A cystoscopy device (100) according to any of the preceding claims, comprising the cavitation probe (300) being made of a material containing polyether ether ketone polymer.

7. A cystoscopy device (100) according to any of the preceding claims, comprising the main probe (200) and the cavitation probe (300) being modular, wherein the outer guide body (211) and the inner guide body (212) have a plurality of tubular body parts joined end to end.

8. A cystoscopy system comprising a cystoscopy device (100) according to any of the preceding claims, comprising a fluid system for transmitting a cavitation fluid to the cavitation probe (300) at a selected pressure and/or at selected time intervals; and an imaging system electrically connected to the image capturing optical unit (400) via the connection cable for displaying the image captured by the image capturing optical unit (400).

9. A cystoscopy system according to claim 8, wherein the fluid system comprises a high-pressure nitrogen tank; a cavitation fluid reservoir; a fluid control valve; a one-way valve; a filter; and a pressure gauge.

## Patentansprüche

1. Eine Zystoskopievorrichtung (100), umfassend eine röhrenförmige Hauptsonde (200); eine Kavitationssonde (300); eine optische Bildaufnahmeeinheit (400), wobei die Hauptsonde (200) nacheinander einen Führungsabschnitt (210), einen Trennabschnitt (220), einen Anschlagabschnitt (230) und einen Gelenkabschnitt (240) mit einer Vielzahl von Ende an Ende ausgerichteten Gelenkeinheiten (241) umfasst;
wobei der Führungsabschnitt (210) mindestens einen rohrförmigen äußeren Führungskörper (211) und mindestens einen rohrförmigen inneren Führungskörper (212) umfasst, der innerhalb des äußeren Führungskörpers (211) angeordnet ist und durch den ein Übertragungsrohr (310) der Kavitationssonde (300) sowie mindestens ein Verbindungskabel verläuft, das elektrisch mit der optischen Bildaufnahmeeinheit (400) verbunden ist; eine Vielzahl von Seilkanälen (213), die sich zwischen dem inneren Führungskörper (212) und dem äußeren Führungskörper (211) entlang des inneren Führungskörpers (212) erstrecken; und einen Adaptereinlass (214) an seinem offenen Ende zur Aufnahme einer tragbaren Steuereinheit (500);
wobei der Trennabschnitt (220) mindestens einen Sondenkanal (250) zur Aufnahme des vom inneren Führungskörpers (212) ausgehenden Übertragungsrohrs (310), mindestens einen Kabelkanal (260) zur Aufnahme des Verbindungskabels sowie eine Vielzahl von Seilkanälen (213) umfasst;
wobei der Anschlagabschnitt (230) dazu dient, während einer Biegebewegung eine Anschlagfläche für die nächstgelegene Gelenkeinheit (241) des Gelenkabschnitts (240) bereitzustellen; wobei jede Gelenkeinheit (241) mindestens einen Sondenkanal (250) und mindestens einen Kabelkanal (260) umfasst; wobei eine der Endflächen der Ende an Ende ausgerichteten Gelenkeinheiten (241) eine innere konkave Form aufweist, die eine Bewegung in nur einem Freiheitsgrad zulässt, und eine andere Endfläche eine äußere konvexe Form aufweist, die der inneren konkaven Fläche entspricht; wobei jede Gelenkeinheit (241), die zwischen zwei Gelenkeinheiten (241) angeordnet ist, mindestens zwei Verbindungszähne (242) aufweist, die sich an einer Stirnfläche einander gegenüberliegend erstrecken, die relative Bewegung der benachbarten Gelenkeinheiten (241) begrenzen und deren Mittelpunkte auf den Mittelpunkt der mittleren Gelenkeinheit (241) ausgerichtet sind; mindestens zwei Zahnausnehmungen (243) an der anderen Endfläche zur Aufnahme und zum Ermöglichen der Bewegung der Verbindungszähne (242) im Inneren; wobei sich eine Vielzahl von Seilzügen entlang der Seilkanäle (213) dem Führungsabschnitt (210), dem Trennabschnitt (220), dem Anschlagabschnitt (230) und dem Gelenkabschnitt (240) erstrecken;
wobei die Kavitationssonde (300) ein flexibles Übertragungsrohr (310) umfasst, das sich entlang der Hauptsonde (200) erstreckt, um ein Kavitationsfluid zu einer strömungsbegrenzende Düse (320) zu leiten; wobei die strömungsbegrenzende Düse (320), die an einem offenen Ende des Gelenkabschnitts (240) angeordnet ist, einen Innenradius aufweist, der kleiner ist als der Innenradius des Übertragungsrohrs (310), um Kavitation zu erzeugen; wobei die optische Bildaufnahmeeinheit (400) am offenen Ende des Gelenkabschnitts (240) angeordnet ist.

2. Eine Zystoskopievorrichtung (100) nach Anspruch 1, umfassend die tragbare Steuereinheit (500) mit einem Sondenadapter (510) zur Aufnahme in den Adaptereinlass (214); wobei die tragbare Steuereinheit (500) mindestens eine Antriebseinheit zum Verändern der Spannung in einer Vielzahl von Seilzügen, die sich entlang der Seilkanäle (213) erstrecken, umfasst, wodurch es dem Gelenkabschnitt (240) ermöglicht wird, sich in nur einem Freiheitsgrad zu biegen und zu bewegen; sowie einen Steuerhebel (520) zum Steuern der Antriebseinheit und damit zum Steuern der Biegebewegung des Gelenkabschnitts (240).

3. Eine Zystoskopievorrichtung (100) nach Anspruch 2, wobei die Antriebseinheit ein Servomotor ist; und mit einem Servoarm, der an einem Ende mit dem Servomotor und am anderen Ende mit den Seilzügen verbunden ist.

4. Eine Zystoskopievorrichtung (100) nach einem der Ansprüche 2 oder 3, umfassend eine Verarbeitungseinheit, die dazu ausgelegt ist, über den Servomotor eine ausgewählte Spannung in den Seilzügen zu erzeugen, um die Position des Gelenkabschnitts (240) in einer durch den Steuerhebel (520) eingestellten Position aufrechtzuerhalten.

5. Eine Zystoskopievorrichtung (100) nach einem der vorstehenden Ansprüche, umfassend eine umgekehrt U-förmige Oberflächenstruktur auf der Oberfläche der Gelenkeinheit (241), die am offenen Ende des Gelenkabschnitts (240) zwischen zwei Seilkanälen (213) angeordnet ist, durch die ein aus einem Seilkanal (312) austretendes Seilzugkabel zum anderen Seilkanal (213) geführt wird.

6. Eine Zystoskopievorrichtung (100) nach einem der vorstehenden Ansprüche, wobei die Kavitationssonde (300) aus einem Material besteht, das ein Polyetheretherketon-Polymer enthält.

7. Eine Zystoskopievorrichtung (100) nach einem der vorstehenden Ansprüche, wobei die Hauptsonde (200) und die Kavitationssonde (300) modular aufgebaut sind, wobei der äußere Führungskörper (211) und der innere Führungskörper (212) eine Vielzahl von rohrförmigen Körperteilen aufweisen, die Ende an Ende miteinander verbunden sind.

8. Ein Zystoskopiesystem, das eine Zystoskopievorrichtung (100) gemäß einem der vorstehenden Ansprüche umfasst, mit einem Fluidsystem zum Zuführen eines Kavitationsfluids zur Kavitationssonde (300) bei einem ausgewählten Druck und/oder in ausgewählten Zeitintervallen; und einem Bildgebungssystem, das über das Verbindungskabel elektrisch mit der optischen Bildaufnahmeeinheit (400) verbunden ist, um das von der optischen Bildaufnahmeeinheit (400) aufgenommene Bild anzuzeigen.

9. Ein Zystoskopiesystem nach Anspruch 8, wobei das Fluidsystem einen Hochdruck-Stickstofftank, einen Kavitationsfluidbehälter, ein Fluidsteuerventil, ein Rückschlagventil, einen Filter und ein Manometer umfasst.

## Revendications

1. Dispositif de cystoscopie (100) comprenant une sonde principale (200) tubulaire ; une sonde de cavitation (300) ; une unité optique de capture d'image (400) ; dans lequel la sonde principale (200) comprend séquentiellement une section de guidage (210) ; une section de séparation (220) ; une section de butée (230) ; et une section articulée (240) présentant une pluralité d'unités d'articulation (241) alignées bout à bout ;
dans lequel la section de guidage (210) comprend au moins un corps de guidage extérieur (211) tubulaire ; au moins un corps de guidage intérieur (212) tubulaire positionné à l'intérieur du corps de guidage extérieur (211), à travers lequel passent un tube de transmission (310) de la sonde de cavitation (300) et au moins un câble de connexion raccordé électriquement à l'unité optique de capture d'image (400) ; une pluralité de canaux de tendon (213) s'étendant entre le corps de guidage intérieur (212) et le corps de guidage extérieur (211) le long du corps de guidage intérieur (212) ; et une entrée d'adaptateur (214) à son extrémité ouverte pour recevoir une unité de commande portable (500) ;
dans lequel la section de séparation (220) comprend au moins un canal de sonde (250) pour recevoir le tube de transmission (310) s'étendant depuis le corps de guidage intérieur (212) ; au moins un canal de câble (260) pour recevoir le câble de connexion ; et une pluralité de canaux de tendon (213) ;
dans lequel la section de butée (230) est destinée à fournir une surface de butée pour l'unité d'articulation (241) la plus proche de la section articulée (240) lors d'un mouvement de flexion ; dans lequel chaque unité d'articulation (241) comprend au moins un canal de sonde (250) et au moins un canal de câble (260) ; dans lequel l'une des surfaces d'extrémité des unités d'articulation (241) alignées bout à bout présente une forme concave intérieure permettant un mouvement selon un seul degré de liberté, et une autre surface d'extrémité présente une forme convexe extérieure pour s'adapter à la surface concave intérieure ; dans lequel chaque unité d'articulation (241) positionnée entre deux unités d'articulation (241) comprend au moins deux dents de raccordement (242) s'étendant en se faisant face sur une surface d'extrémité, limitant le mouvement relatif des unités d'articulation (241) adjacentes et dont le centre fait face au centre de l'unité d'articulation (241) centrale ; au moins deux évidements pour dents (243) sur l'autre surface d'extrémité pour recevoir les dents de raccordement (242) et permettre leur mouvement à l'intérieur ; dans lequel une pluralité de câbles de tendon s'étendent le long des canaux de tendon (213) de la section de guidage (210), de la section de séparation (220), de la section de butée (230) et de la section articulée (240) ;
dans lequel la sonde de cavitation (300) comprend un tube de transmission (310) flexible s'étendant le long de la sonde principale (200) pour transmettre un fluide de cavitation à une buse de restriction de débit (320) ; la buse de restriction de débit (320) étant positionnée à une extrémité ouverte de la section articulée (240) et présentant un rayon intérieur inférieur au rayon intérieur du tube de transmission (310) pour créer une cavitation ; dans lequel l'unité optique de capture d'image (400) est positionnée à l'extrémité ouverte de la section articulée (240).

2. Dispositif de cystoscopie (100) selon la revendication 1, comprenant l'unité de commande portable (500) ayant un adaptateur de sonde (510) destiné à être logé dans l'entrée d'adaptateur (214) ; dans lequel l'unité de commande portable (500) comprend au moins une unité d'entraînement pour modifier la tension dans une pluralité de câbles de tendon s'étendant le long des canaux de tendon (213) et permettant ainsi à la section articulée (240) de se fléchir et de se déplacer selon un seul degré de liberté ; et un levier de commande (520) pour commander l'unité d'entraînement et commander ainsi le mouvement de flexion de la section articulée (240).

3. Dispositif de cystoscopie (100) selon la revendication 2, dans lequel l'unité d'entraînement est un servomoteur ; et comprenant un bras de servomoteur relié au servomoteur à une extrémité et aux câbles de tendon à l'autre extrémité.

4. Dispositif de cystoscopie (100) selon l'une quelconque des revendications 2 ou 3, comprenant une unité de traitement adaptée pour créer une tension sélectionnée dans les câbles de tendon via le servomoteur afin de maintenir la position de la section articulée (240) dans une position réglée par le levier de commande (520).

5. Dispositif de cystoscopie (100) selon l'une quelconque des revendications précédentes, comprenant une structure de surface en forme de U inversé sur la surface de l'unité d'articulation (241) positionnée à l'extrémité ouverte de la section articulée (240), entre deux canaux de tendon (213), à travers laquelle un câble de tendon sortant d'un canal de tendon (312) est guidé vers l'autre canal de tendon (213).

6. Dispositif de cystoscopie (100) selon l'une quelconque des revendications précédentes, comprenant la sonde de cavitation (300) constituée d'un matériau contenant un polymère de polyétheréthercétone.

7. Dispositif de cystoscopie (100) selon l'une quelconque des revendications précédentes, comprenant la sonde principale (200) et la sonde de cavitation (300) qui sont modulaires, dans lequel le corps de guidage extérieur (211) et le corps de guidage intérieur (212) comportent une pluralité de parties de corps tubulaires reliées bout à bout.

8. Système de cystoscopie comprenant un dispositif de cystoscopie (100) selon l'une quelconque des revendications précédentes, comprenant un système fluidique pour transmettre un fluide de cavitation à la sonde de cavitation (300) à une pression sélectionnée et/ou à des intervalles de temps sélectionnés ; et un système d'imagerie raccordé électriquement à l'unité optique de capture d'image (400) via le câble de connexion pour afficher l'image capturée par l'unité optique de capture d'image (400).

9. Système de cystoscopie selon la revendication 8, dans lequel le système fluidique comprend un réservoir d'azote haute pression ; un réservoir de fluide de cavitation ; une vanne de régulation de fluide ; un clapet antiretour ; un filtre ; et un manomètre.
